# EUROPÄISCHE PATENTSCHRIFT

(11) **EP 1 263 746 B1**
(45) Veröffentlichungstag und Bekanntmachung des Hinweises auf die Patenterteilung: **28.12.2005**
(21) Anmeldenummer: 01903742.3
(22) Anmeldetag: 12.02.2001
(51) Int. Cl.: C07D 277/60, A61K 31/425

(54) **SUBSTITUIERTE 8,8a-DIHYDRO-3aH-INDENO 1,2-d]THIAZOLE, VERFAHREN ZU IHRER HERSTELLUNG UND IHRE VERWENDUNG ALS ARZNEIMITTEL**
SUBSTITUTED 8, 8A-DIHYDRO-3AH-INDENO 1,2-D]THIAZOLES, A METHOD FOR THEIR PRODUCTION AND THEIR USE AS MEDICAMENTS
8,8A-DIHYDRO-3AH-INDENO 1,2-D]THIAZOLES SUBSTITUES, PROCEDES DE FABRICATION ET UTILISATION EN TANT QU'AGENTS PHARMACEUTIQUES

(30) Priorität: 23.02.2000 DE 10008274
(43) Veröffentlichungstag der Anmeldung: 11.12.2002
(73) Patentinhaber: Sanofi-Aventis Deutschland GmbH, 65929 Frankfurt am Main (DE)
(72) Erfinder: JAEHNE, Gerhard, 65929 Frankfurt (DE); LANG, Hans-Jochen, 65719 Hofheim (DE); GOSSEL, Matthias, 65719 Hofheim (DE); BICKEL, Martin, 61348 Bad Homburg (DE)
(86) Internationale Anmeldenummer: PCT/EP2001/001498
(87) Internationale Veröffentlichungsnummer: WO 2001/062745

(56) Entgegenhaltungen:
- EP-A- 0 749 966
- WO-A-00/18749
- WO-A-00/51994
- FR-A- 2 338 937
- FR-A- 2 364 219
- US-A- 3 507 868

## Beschreibung

Substituierte 8,8a-Dihydro-3aH-indeno[1,2-d]thiazole, Verfahren zu ihrer Herstellung und ihre Verwendung als Arzneimittel

Die Erfindung betrifft substituierte 8,8a-Dihydro-3aH-indeno[1,2-d]thiazole sowie deren physiologisch verträgliche Salze und physiologisch funktionelle Derivate.

Es sind bereits Thiazolidinderivate mit anorektischer Wirkung im Stand der Technik beschrieben (Österreichisches Patent Nr. 365181).

Der Erfindung lag die Aufgabe zugrunde, weitere Verbindungen zur Verfügung zu stellen, die eine therapeutisch verwertbare anorektische Wirkung entfalten.

Die Erfindung betrifft daher Verbindungen der Formel I, 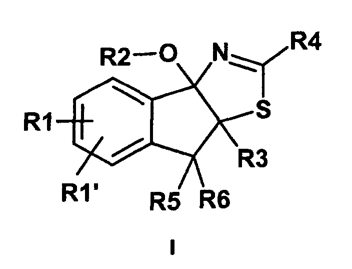 worin bedeuten
- R1, R1': unabhängig voneinander H, F, Cl, Br, -OH, O-(C₁-C₆)-Alkyl, (C₁-C₆)-Alkyl, wobei in den Alkylresten ein Wasserstoff durch OH ersetzt sein kann;
- R2: H, (C₁-C₆)-Alkyl, C(O)-(C₁-C₆)-Alkyl;
- R3: Cl, Br, (CH₂)₀₋₁-COO(C₁-C₆)-Alkyl, (CH₂)₀₋₁-COOH, (CH₂)₀₋₁-CONH₂;
- R4: (C₁-C₄)-Alkyl oder (C₃-C₆)-Cycloalkyl, wobei in den Alkylresten ein Wasserstoff durch OH ersetzt sein kann;
(CH₂)₀₋₆-Aryl, wobei Aryl gleich Phenyl, 1- oder 2-Naphthyl, 2-, 3- oder 4-Pyridyl, 2- oder 3-Thienyl sein kann und der Arylrest bis zu zweifach mit F, Cl, Br, OH, CF₃, O-(C₁-C₆)-Alkyl, SO₂-(C₁-C₆)-Alkyl, (CH₂)₀₋₆-SO₂-NH₂, (C₁-C₆)-Alkyl, COOH, COO(C₁-C₆)Alkyl oder CONH₂ substituiert sein kann;
(CH₂)₁₋₆-A-R8;
- A: O, SO₂;
- R8: (C₁-C₈)-Alkyl, (C₃-C₈)-Cycloalkyl, wobei in den Alkylresten ein Wasserstoff durch OH ersetzt sein kann;
(CH₂)₀₋₆-Aryl, wobei Aryl gleich Phenyl oder Thienyl sein kann und der Arylteil bis zu zweifach mit F, Cl, Br, OH, CF₃, O-(C₁-C₆)-Alkyl, SO₂-(C₁-C₆)-Alkyl, SO₂-NH₂, COOH, COO(C₁-C₆)Alkyl oder CONH₂ sein kann;
- R5: H
- R6: OH, O-(C₁-C₆)-Alkyl, O-C(O)-(C₁-C₆)-Alkyl, O-C(O)-Aryl, wobei Aryl gleich Phenyl oder Thienyl sein kann; O-alpha oder beta-Glucuronsäure;
SO₂-(C₁-C₆)-Alkyl, SO₂-(CH₂)₀₋₆-Phenyl, NH₂, Morpholin-4-yl, NH-C(O)-(C₁-C₆)-Alkyl, NH-C(O)-Phenyl
oder
R5 und R6 bilden zusammen =O oder -O-(CH₂)₂₋₆-O-,
sowie deren physiologisch verträgliche Salze

Die Erfindung bezieht sich auf Verbindungen der Formel I, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R1', R2, R3, R4, R6, R8 und A können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-,
Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

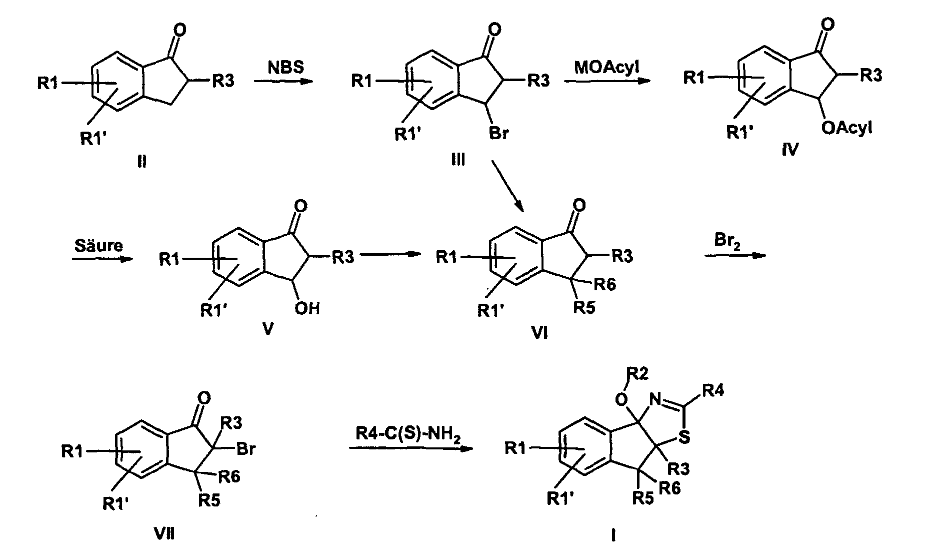

Dazu werden Verbindungen der allgemeinen Formel II, 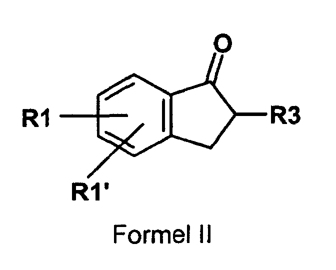 worin R1, R1' und R3 'die angegebene Bedeutung besitzen, mit N-Bromsuccinimid in eine Verbindung der Formel III überführt.
Die Verbindungen der Formel III werden weiter mit Metallsalzen organischer Säuren (MOAcyl), wie z.B. Silberacetat, in Verbindungen der Formel IV überführt. Andererseits kann man Verbindungen der Formel III durch nukleophilen Austausch mit O-, S- oder N-Nukleophilen in die entsprechenden O-, S- oder N-substituierten Verbindungen der Formel VI, worin die Reste R1, R1', R3, R5 und R6 die beschriebene Bedeutung haben, umwandeln.
Verbindungen der Formel IV können durch z.B. saure Hydrolyse in Verbindungen der Formel V überführt werden.
Die Verbindungen der Formeln V und VI können unter Standardbedingungen durch Reaktion mit Brom in die entsprechenden alpha-Brom-Ketone der Formel VII, worin z.B. R5 gleich Wasserstoff und R6 gleich OH sein kann, überführt werden.
Der Umsatz der Thioamide der Formel R4-C(S)-NH₂, worin R4 die vorn beschriebenen Bedeutungen hat, mit Verbindungen der allgemeinen Formel VII führt zu Verbindungen der allgemeinen Formel I, worin R2 gleich Wasserstoff ist. Diese Verbindungen können unter Einsatz von Standardmethoden in Verbindungen der Formel I, worin R2 die weiter vorn beschriebenen Bedeutungen hat, umgesetzt werden.

Zur Salzbildung kommen als anorganische Säuren beispielsweise in Betracht:
Halogenwasserstoffsäuren wie Chlorwasserstoffsäure und Bromwasserstoffsäure, sowie Schwefelsäure, Phosphorsäure und Amidosulfonsäure.

Als organische Säuren zur Salzbildung seien beispielsweise genannt:
Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen der Formel VII mit den Thioamiden R4-C(S)-NH₂ im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dimethylsulfoxid, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen - 10°C und 150°C, bevorzugt zwischen 30°C und 100°C, durchgeführt werden. Als besonders günstig erwies sich in der Regel ein Temperaturbereich zwischen 50°C und 90°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen I in Form ihrer Säureadditionssalze im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen I, ggf. nach Konzentrierung der Reaktionslösung, mit einem der genannten Fällungsmittel ausfällen. Ferner kann man die Lösung des Reaktionsgemisches auch vorteilhaft in die Lösung eines der genannten Fällungsmittel unter Rühren einfiltrieren. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Verbindung der Formel I ein Säureadditionsprodukt der Formel I so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisiopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.
Die Reaktion der Verbindungen der Formel VII mit den Thioamiden R4-C(S)-NH₂ kann auch so durchgeführt werden, daß man zur Reaktionsmischung eine wenigstens äquimolare Menge einer Base, wie z.B. Triethylamin, zufügt und die so erhaltenen freien Basen der Formel I anschließend gegebenenfalls in ihre Säureadditionsprodukte überführt.

Die Säureadditionsprodukte der Verbindungen der Formel I können durch Behandlung mit Basen zu den freien Basen der allgemeinen Formel I umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Thioamide der allgemeinen Formel R4-C(S)-NH₂ sind entweder kommerziell erhältlich oder können z.B. durch Umsetzung des entsprechenden Carbonsäureamids mit Phosphorpentasulfid in Pyridin (R. N. Hurd, G. Delameter, Chem. Rev. 61, 45 (1961)), oder mit Lawesson's Reagenz in Toluol, Pyridin, Hexamethylphosphorsäurtriamid [Scheibye, Pedersen und Lawesson: Bull. Soc. Chim. Belges 87, 229 (1978)], vorzugsweise in einem Gemisch von Tetrahydrofuran mit 1,3-Dimethyl-3,4,5,6-tetrahydro-2(1H)-pyrimidinon oder 1,3-Dimethyl-2-Imidazolidinon gewonnen werden. Hydroxy-, Amino- oder zusätzliche Carbonylfunktionen werden dabei zweckmäßigerweise mit einer wiederabspaltbaren Schutzfunktion, wie z. B. einem Benzyl-, tert.Butyloxycarbonyl-, Benzyloxycarbonylrest bzw. durch Überführung in ein, gegebenfalls cyclisches, Acetal überführt. Methoden dazu sind z. B. beschrieben in Th. W. Greene und P. G. M. Wuts, Protective Groups in Organic Synthesis, Second Edition, 1991, John Wiley & Sons, New York.

Thioamide der Formel R4-C(S)-NH₂ sind auch erhältlich, indem man Nitrile der allgemeinen Formel R4-CN mit Schwefelwasserstoff (Houben-Weyl IX, 762) oder Thioacetamid (E. C. Taylor, J. A. Zoltewicz, J. Am. Chem. Soc. 82, 2656 (1960)) oder O,O-Diethyl-dithiophosphorsäure umsetzt. Die Umsetzungen mit Schwefelwasserstoff werden vorzugsweise in einem organischen Lösemittel wie Methanol oder Ethanol, die mit Thioacetamid in einem Lösemittel wie Dimethylformamid unter Hinzufügung von Salzsäure, die mit O,O-Diethyldithiophosphorsäure in einem Lösemittel wie Essigsäureethylester unter sauren, z.B. HCl, Bedingungen bei Raumtemperatur oder unter Erwärmung durchgeführt.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

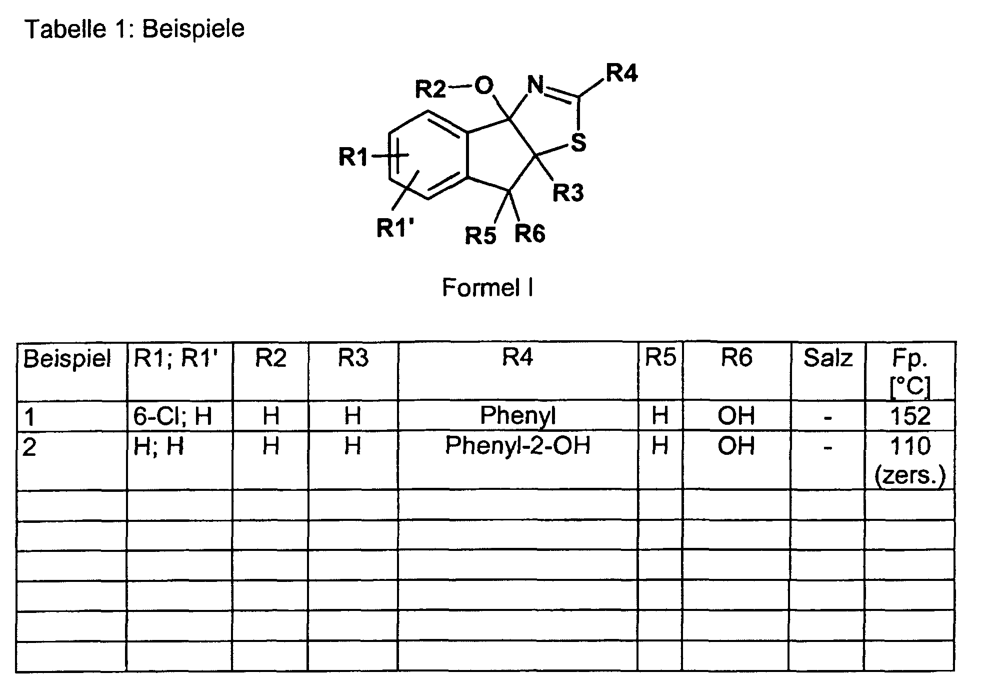

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

Die Wirksamkeit der Verbindungen wurde wie folgt getestet:

### Biologisches Prüfmodell:

Die Prüfung der anorektischen Wirkung erfolgte an weiblichen NMRI Mäusen.
Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 1,5 Stunden lang bestimmt und das Allgemeinbefinden der Tiere beobachtet. Der gemessene Milchverbrauch wurde mit dem unbehandelter Kontrolltieren verglichen.

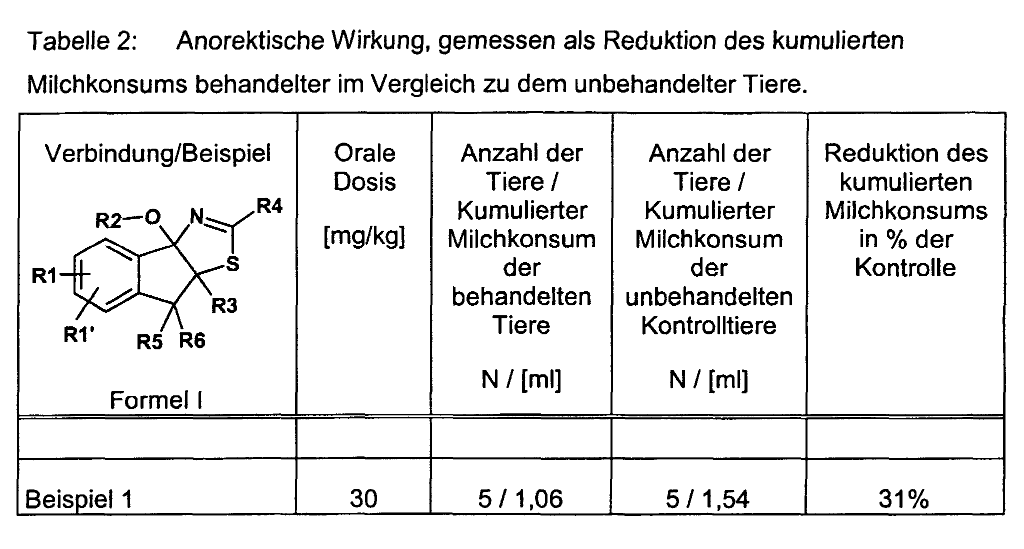

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1 (Verbindung 1):

6-Chlor-2-phenyl-8,8a-dihydro-indeno[1,2-d]thiazol-3a,8-diol:
a) 3-Brom-5-chlor-indan-1-on:
   8.33 g (50 mmol) 5-Chlor-indan-1-on wurden mit 8.9 g (50 mmol) N-Bromsuccinimid in 175 ml Tetrachlorkohlenstoff suspendiert, mit 1 g Benzoylperoxid vesetzt und unter Rühren 3 h zum Rückfluß erhitzt. Die abgekühlte Reaktionslösung wurde filtriert, 2 x mit 100 ml Wasser ausgeschüttelt, über Magnesiumsulfat getrocknet, filtriert und im Vakuum eingeengt. Der Rückstand wurde in 120 ml einer 1:1 Mischung aus n-Heptan und Cyclohexan heiß gelöst, mit Aktivkohle aufgekocht, filtriert und das Filtrat im Vakuum eingeengt. Man erhält 3-Brom-5-chlor-indan-1-on mit dem Schmelzpunkt 96-97°C
b) 3-Acetoxy-5-chlor-indan-1-on:
   4.91 g (20 mmol) 3-Brom-5-chlor-indan-1-on wurden mit 3.34 (20 mmol) Silberacetat in 100 ml Essigsäure suspendiert und unter Rühren 5 h zum Rückfluß erhitzt. Die abgekühlte Reaktionslösung wurde im Vakuum eingeengt und der Rückstand über Kieselgel mit Toluol/Aceton 10/1 chromatographisch gereinigt. Man erhielt 3-Acetoxy-5-chlor-indan-1-on mit dem Schmelzpunkt 65-67°C.
c) 5-Chlor-3-hydroxy-indan-1-on:
   1.98 g (8.8 mmol) 3-Acetoxy-5-chlor-indan-1-on wurden in 10 ml Acetonitril unter Zugabe von 50 ml 3N HCl gelöst. Die Reaktionsmischung wurde 48 h bei Raumtemperatur gerührt. Danach wurde das Lösemittel Acetonitril im Vakuum abdestilliert, und das ausgefallene Rohprodukt wurde abfiltriert und über Kieselgel mit Toluol/Aceton 5/1 chromatographisch gereinigt. Das Produkt, 5-Chlor-3-hydroxy-indan-1-on, schmolz bei 125-128°C.
d) 2-Brom-5-chlor-3-hydroxy-indan-1-on:
   0.69 g (3.78 mmol) 5-Chlor-3-hydroxy-indan-1-on wurden in 100 ml Diethylether gelöst. Unter Rühren wurde 1 Tropfen Brom zugegeben und bis zur Entfärbung gerührt. Danach wurde auf -5°C abgekühlt und 0.194 ml (3.78 mmol) Brom in 2 ml Dichlormethan innerhalb von 30 min. zugetropft. Anschließend wurde noch 30 min. gerührt, mit 50 ml Wasser versetzt, die organische Phase abgetrennt, über Magnesiumsulfat getrocknet und filtriert. Das Filtrat wurde im Vakuum eingeengt und der Rückstand im Vakuum getrocknet. Man erhielt 2-Brom-5-chlor-3-hydroxy-indan-1-on mit dem Schmelzpunkt 118-119°C.
e) 6-Chlor-2-phenyl-8,8a-dihydro-indeno[1,2-d]thiazol-3a,8-diol:
   0.6 g (2.3 mmol) 2-Brom-5-chlor-3-hydroxy-indan-1-on wurden mit 0.473 g (3.45 mmol) Thiobenzamid in 5 ml Isopropanol gelöst. Nach Zugabe von 0.478 ml (3.45 mmol) Triethylamin wurde 12 h bei Raumtemperatur und anschließend noch 8 h bei 50°C gerührt. Die abgekühlte Reaktionsmischung wurde im Vakuum eingeengt und der Rückstand über Kieselgel erst mit n-Heptan/Essigsäureethylester 3/1 und anschließend mit Toluol/Aceton 5/1 chromatographisch gereinigt. Man erhielt 6-Chlor-2-phenyl-8,8a-dihydro-indeno[1,2-d]thiazol-3a,8-diol mit dem Schmelzpunkt (unter Zersetzung) von 151-153°C.

## Patentansprüche

1. Verbindungen der Formel I, 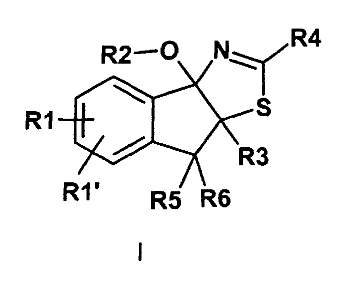 worin bedeuten
R1, R1' unabhängig voneinander H, F, Cl, Br, -OH, O-(C₁-C₆)-Alkyl, (C₁-C₆)-Alkyl, wobei in den Alkylresten ein Wasserstoff durch OH ersetzt sein kann;
R2 H, (C₁-C₆)-Alkyl, C(O)-(C₁-C₆)-Alkyl;
R3 Cl, Br, (CH₂)₀₋₁-COO(C₁-C₆)-Alkyl, (CH₂)₀₋₁-COOH, (CH₂)₀₋₁-CONH₂;
R4 (C₁-C₄)-Alkyl oder (C₃-C₆)-Cycloalkyl, wobei in den Alkylresten ein Wasserstoff durch OH ersetzt sein kann;
(CH₂)₀₋₆-Aryl, wobei Aryl gleich Phenyl, 1- oder 2-Naphthyl, 2-, 3- oder 4-Pyridyl, 2- oder 3-Thienyl sein kann und der Arylrest bis zu zweifach mit F, Cl, Br, OH, CF₃, O-(C₁-C₆)-Alkyl, SO₂-(C₁-C₆)-Alkyl, (CH₂)₀₋₆-SO₂-NH₂, (C₁-C₆)-Alkyl, COOH, COO(C₁-C₆)Alkyl oder CONH₂ substituiert sein kann;
(CH₂)₁₋₆-A-R8;
A O, SO₂;
R8 (C₁-C₈)-Alkyl, (C₃-C₈)-Cycloalkyl, wobei in den Alkylresten ein Wasserstoff durch OH ersetzt sein kann;
(CH₂)₀₋₆-Aryl, wobei Aryl gleich Phenyl oder Thienyl sein kann und der Arylteil bis zu zweifach mit F, Cl, Br, OH, CF₃, O-(C₁-C₆)-Alkyl, SO₂-(C₁-C₆)-Alkyl, SO₂-NH₂, COOH, COO(C₁-C₆)Alkyl oder CONH₂ sein kann;
R5 H
R6 OH, O-(C₁-C₆)-Alkyl, O-C(O)-(C₁-C₆)-Alkyl, O-C(O)-Aryl, wobei Aryl gleich Phenyl oder Thienyl sein kann; O-alpha oder beta-Glucuronsäure;
SO₂-(C₁-C₆)-Alkyl, SO₂-(CH₂)₀₋₆-Phenyl, NH₂, Morpholin-4-yl, NH-C(O)-(C₁-C₆)-Alkyl, NH-C(O)-Phenyl
oder
R5 und R6 bilden zusammen =O oder -O-(CH₂)₂₋₆-O-,
sowie deren physiologisch verträgliche Salze.

2. Arzneimittel enthaltend eine oder mehrere der Verbindungen gemäß Anspruch 1.

3. Verbindungen gemäß Anspruch 1 zur Anwendung als Medikament zur Prophylaxe oder Behandlung der Obesitas.

4. Verbindungen gemäß Anspruch 1 zur Anwendung als Medikament zur Prophylaxe oder Behandlung des Typ II Diabetes.

5. Verbindungen gemäß Anspruch 1 in Kombination mit mindestens einem weiteren anorektischen Wirkstoff zur Anwendung als Medikament zur Prophylaxe oder Behandlung der Obesitas.

6. Verbindungen gemäß Anspruch 1 in Kombination mit mindestens einem weiteren anorektischen Wirkstoff zur Anwendung als Medikament zur Prophylaxe oder Behandlung der des Typ II Diabetes.

7. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

8. Verwendung der Verbindungen gemäß Anspruch 1 zur Herstellung eines Medikaments zur Prophylaxe oder Behandlung der Obesitas.

9. Verwendung der Verbindungen gemäß Anspruch 1 zur Herstellung eines Medikaments zur Prophylaxe oder Behandlung des Typ II Diabetes.

10. Verfahren zur Herstellung der Verbindungen gemäß Anspruch 1, **dadurch gekennzeichnet, daß** man nach folgendem Formelschema 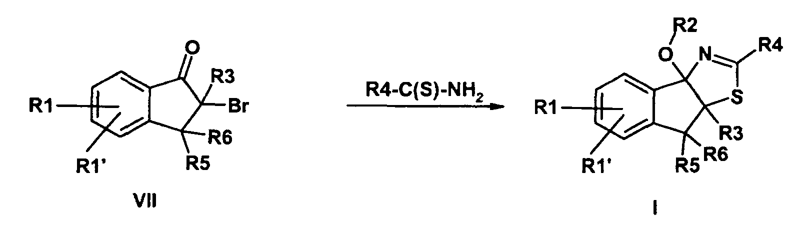 eine Verbindung der Formel VII, worin die Reste die zu Formel I angegebenen Bedeutungen haben mit einem Thioamid der Formel R4-C(S)-NH₂, worin R4 die zu Formel I angegebene Bedeutung hat, zu Verbindungen der allgemeinen Formel I, worin R2 gleich Wasserstoff ist umsetzt und diese gegebenenfalls an R2 Position so weiter derivatisiert, daß die übrigen zu Formel I angegebenen Reste R2 entstehen.

## Claims

1. A compound of the formula I, 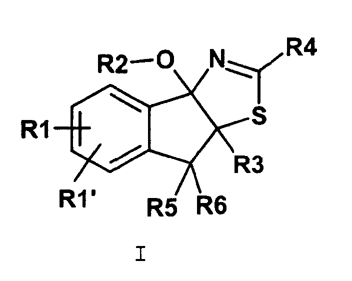 in which
R1, R1' independently of one another are H, F, Cl, Br, -OH, O-(C₁-C₆)-alkyl, (C₁-C₆)-alkyl, where in the alkyl radicals one hydrogen can be replaced by OH;
R2 is H, (C₁-C₆)-alkyl, C(O)-(C₁-C₆)-alkyl;
R3 is Cl, Br, (CH₂)₀₋₁-COO(C₁-C₆)-alkyl, (CH₂)₀₋₁-COOH, (CH₂)₀₋₁-CONH₂;
R4 is (C₁-C₄)-alkyl or (C₃-C₆)-cycloalkyl, where in the alkyl radicals one hydrogen can be replaced by OH;
(CH₂)₀₋₆-aryl, where aryl can be phenyl, 1- or 2-naphthyl, 2-, 3- or 4-pyridyl, 2- or 3-thienyl and the aryl radical can be substituted up to two times by F, Cl, Br, OH, CF₃, O-(C₁-C₆)-alkyl, SO₂-(C₁-C₆)-alkyl, (CH₂)₀₋₆-SO₂-NH₂; (C₁-C₆)-alkyl, COOH, COO (C₁-C₆)-alkyl or CONH₂;
(CH₂)₁₋₆-A-R8;
A is O, SO₂;
R8 is (C₁-C₈)-alkyl, (C₃-C₈) -cycloalkyl, where in the alkyl radicals one hydrogen can be replaced by OH;
(CH₂)₀₋₆-aryl, where aryl can be phenyl or thienyl and the aryl moiety can be substituted up to two times by F, Cl, Br, OH, CF₃, O-(C₁-C₆)-alkyl, SO₂-(C₁-C₆)-alkyl, SO₂-NH₂, COOH, COO(C₁-C₆) -alkyl or CONH₂;
R5 is H;
R6 is OH, O-(C₁-C₆)-alkyl, O-C(O)-(C₁-C₆)-alkyl, O-C(O)-aryl, where aryl can be phenyl or thienyl; O-alpha or -beta-glucuronic acid;
SO₂-(C₁-C₆)-alkyl, SO₂-(CH₂)₀₋₆-phenyl, NH₂, morpholin-4-yl, NH-C(O)-(C₁-C₆)-alkyl, NH-C(O)-phenyl
or
R5 and R6 together form =O or -O-(CH₂)₂₋₆-O-,
and its physiologically acceptable salts.

2. A medicament, comprising one or more compounds as claimed in claim 1.

3. A compound as claimed in claim 1 for use as a medicament for the prophylaxis or treatment of obesity.

4. A compound as claimed in claim 1 for use as a medicament for the prophylaxis or treatment of type II diabetes.

5. A compound as claimed in claim 1 in combination with at least one further anorectic for use as a medicament for the prophylaxis or treatment of obesity.

6. A compound as claimed in claim 1 in combination with at least one further anorectic for use as a medicament for the prophylaxis or treatment of type II diabetes.

7. A process for preparing a medicament comprising one or more of the compounds as claimed in claim 1, which comprises mixing the active compound with a pharmaceutically suitable excipient and bringing this mixture into a form suitable for administration.

8. The use of the compounds as claimed in claim 1 for preparing a medicament for the prophylaxis or treatment of obesity.

9. The use of the compounds as claimed in claim 1 for preparing a medicament for the prophylaxis or treatment of type II diabetes.

10. A process for preparing compounds as claimed in claim 1, which comprises reacting, according to the equation below, 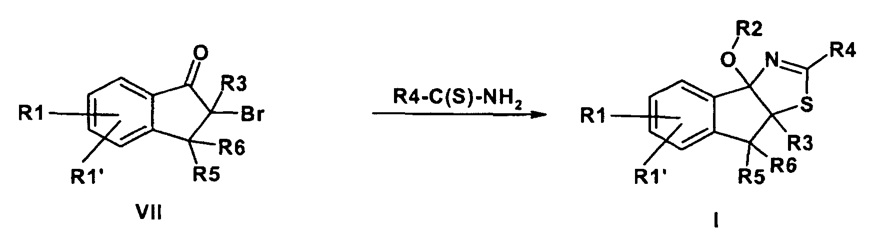 a compound of the formula VII in which the radicals are as defined for formula I with a thioamide of the formula R4-C(S)-NH₂, in which R4 is as defined for formula I to give compounds of the formula I in which R2 is hydrogen and, if appropriate, derivatizing these compounds further at the R2 position such that the other radicals R2 given to formula I are formed.

## Revendications

1. Composés de formule I, 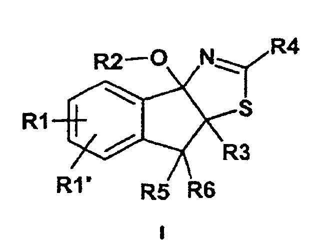 dans laquelle R1, R1' représentent, indépendamment l'un de l'autre, H, F, Cl, Br, -OH, O-(C₁-C₆)-alkyle, un aklyle en (C₁-C₆), où dans les radicaux alkyles, un hydrogène peut être remplacé par OH ;
R2 représente H, un alkyle en (C₁-C₆), un C(O)-(C₁-C₆)-alkyle ;
R3 représente Cl, Br, (CH₂)₀₋₁-COO(C₁-C₆)-alkyle, (CH₂)₀₋₁-COOH, (CH₂)₀₋₁-CONH₂ ;
R4 représente un alkyle en (C₁-C₄) ou un cycloalkyle en (C₃-C₆), où dans les radicaux alkyles, un hydrogène peut être remplacé par OH ;
Un (CH₂)₀₋₆-aryle, dans lequel l'aryle peut être un phényle, un 1- ou un 2- naphtyle, un 2-, 3- ou un 4-pyridyle, un 2- ou un 3-thiényle et le radical aryle peut être substitué jusqu'à deux fois par F, Cl, Br, OH, CF₃, O-(C₁-C₆)-alkyle, un SO₂-(C₁-C₆)-alkyle, (CH₂)₀₋₆-SO₂-NH₂, un alkyle en (C₁-C₆), COOH, un COO(C₁-C₆)-alkyle ou CONH₂ ;
(CH₂)₁₋₆-A-R8 ;
A représente O, SO2 ;
R8 représente un alkyle en (C₁-C₈), un cycloalkyle en (C₃-C₈) où dans les radicaux alkyles, un hydrogène peut être remplacé par OH ;
(CH₂)₀₋₆-aryle, dans lequel l'aryle peut être un phényle ou un thiényle et la partie aryle peut être substituée jusqu'à deux fois par F, Cl, Br, OH, CF₃, un O-(C₁-C₆)-alkyle, un SO₂-(C₁-C₆)-alkyle, SO₂-NH₂, COOH, un COO(C₁-C₆)-alkyle ou CONH₂;
R5 représente H ;
R6 représente OH, un O-(C₁-C₆)-alkyle, un O-C(O)-(C₁-C₆)-alkyle, un O-C(O)-aryle, dans lequel l'aryle peut être un phényle ou un thyényle ; un acide O-alpha ou beta-glucuronique ;
un SO₂-(C₁-C₆)-alkyle, SO₂-(CH₂)₀₋₆-phényle, NH₂, un morpholin-4-yle, un NH-C(O)-(C₁-C₆)-alkyle, un NH-C(O)-phényle
ou
R5 et R6 forment conjointement =O ou -O-(CH₂)₂₋₆-O-,
ainsi que leurs sels physiologiquement acceptables.

2. Agent pharmaceutique contenant un ou plusieurs des composés selon la revendication 1.

3. Composés selon la revendication 1, destinés à être utilisés en tant que médicament pour la prophylaxie ou le traitement de l'obésité.

4. Composés selon la revendication 1, destinés à être utilisés en tant que médicament pour la prophylaxie ou le traitement du diabète de type II.

5. Composés selon la revendication 1 en combinaison avec au moins un ingrédient actif anorexiant supplémentaire, destinés à être utilisés en tant que médicament pour la prophylaxie ou le traitement de l'obésité.

6. Composés selon la revendication 1 en combinaison avec au moins un ingrédient actif anorexiant supplémentaire, destinés à être utilisés en tant que médicament pour la prophylaxie ou le traitement du diabète de type II.

7. Procédés de production d'un agent pharmaceutique contenant un ou plusieurs des composés selon la revendication 1, **caractérisé en ce que** l'ingrédient actif est mélangé avec un support pharmaceutiquement approprié et ce mélange est mis sous une forme appropriée pour l'administration.

8. Utilisation des composés selon la revendication 1 pour la production d'un médicament destiné à la prophylaxie ou au traitement de l'obésité.

9. Utilisation des composés selon la revendication 1 pour la production d'un médicament destiné à la prophylaxie ou au traitement du diabète de type II.

10. Procédés de préparation des composés selon la revendication 1, **caractérisés en ce que**, conformément au schéma de formules suivant 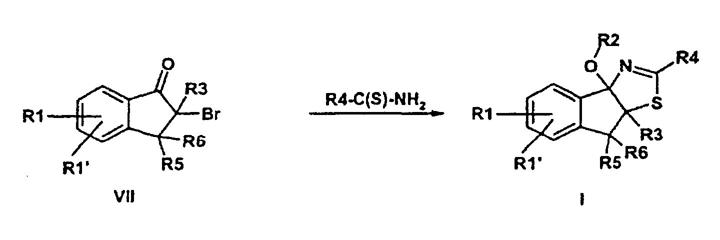 un composé de formule VII, dans laquelle les radicaux présentent les significations indiquées pour la formule I, est mis à réagir avec un thioamide de formule R4-C(S)-NH₂, dans laquelle R4 présente la signification indiquée pour la formule I, en donnant lieu à des composés de formule générale I, dans laquelle R2 est un hydrogène, et ceux-ci sont éventuellement davantage dérivatisés au niveau de la position R2 de telle sorte que les autres radicaux R2 indiqués pour la formule I soient formés.
